# EUROPEAN PATENT APPLICATION

(11) **EP 3 130 373 A1**
(43) Date of publication of application: **15.02.2017**
(21) Application number: 16179325.2
(22) Date of filing: 13.07.2016
(51) Int. Cl.: A61N 1/04, A61N 1/32

(54) **A DEVICE FOR STIMULATING IN THE HUMAN BODY THE PRODUCTION OF NITRIC OXIDE**

(30) Priority: 31.07.2015 IT UB20152732
(71) Applicant: Akern S.r.L., 50065 Pontassieve (FI) (IT)
(72) Inventor: TALLURI, Antonio, 50012 Bagno a Ripoli (FI) (IT)
(74) Representative: Emmi, Mario

(57) **Abstract**

In accordance with the invention, it is described the possibility of being able to stimulate in the human body the production of nitric oxide through a device that foresees the arrangement of a generator and of a conductive support surface (2) connected to the generator.

The generator is made to operate within a range comprised between 5Kv and 7Kv with a circulating current comprised between 0,1mA and 0,5mA and with a frequency between 50 and 60 Hz.

The pre-set values foresee a current at a value of 0,1mA and with a voltage of 5000 Volts and frequency of 50 hertz.

## Description

### Technical field

The present invention concerns the technical field relative to the medical instruments whose functioning is based on the generation of electrostatic fields.

In particular, the invention refers to a generator of electrostatic field, and relative ionizing mat to which it is connected, which allow and stimulate in the organism the production of nitric oxide.

### Background art

The beneficial effects of the nitric oxide (NO) have been known for some years.

For instance, Nobel prize winner Louis Ignarro obtained the prize in 1998 precisely for such a discovery and has published in this regard various scientific texts, among which the following:
"No More Heart Disease: How Nitric Oxide Can Prevent---Even Reverse---Heart Disease and Stroke".

The beneficial effects of the nitric oxide (NO) are mainly directed to the cardiovascular system, which results more resistant and more protected from ageing effects.

At the current state of the art, however, the production of nitric oxide can be supplied to the body artificially, just through the use of specific drugs, among which those containing L-Arginine and L-Citrulline.

Naturally, the use of chemical substances is not always advised, because these can have contraindications, can produce undesired collateral effects and can give place to various health problems. Further, it is not always obvious that a drug can be used by anyone. In fact, if a subject suffers from a particular pathology it is not obvious that he can use said drug without incurring also serious health risks.

Even if some devices of electric nature are known, these have been used and adjusted in order to solve pathologies of different nature without ever being capable, any of them, of improving or favouring the production of nitric oxide.

It is known, for example, a device called New Healt 9000, discussed in publication MARIO LIANI "Effects of a pulsatile electrostatic field on ischemic injury to the diabetic foot: Evaluation of refractory ulcers", Vol. 8 n° 3,1, which is a machinery that, above all, is cited again also in the body of the description of the present invention.

In such a publication, the use of such New Healt 9000 machinery is in fact described to make a pulsating electrostatic treatment introducing negative charges, with a voltage comprised between 2000 V and 9000 V with a low pulsating current. The aim described in the publication, and the relative programming parameters discussed, are however finalized at modifying the electric charge present on the membrane surface of the blood cells and therefore improving the vasomotor system in obese and diabetic subjects. Basically, such a system optimizes the oxygenation in the organism. Nevertheless, as also introduced in the part of description of the present invention, such a machinery, if operating in said ranges of voltage and current described in said publication, is not capable of stimulating any production of nitric oxide but is able to improve exclusively the oxygenation for other different purposes.

Likewise, publication A De Lorenzo: "Resting metabolic rate incremented by pulsating electrostatic field (PESF) therapy" resumes again and discusses the use of the same New Healt 9000 machinery mainly for the same aim, to improve the oxygenation of the tissues. In De Lorenzo the adjustment of voltage to various possible values of voltage is suggested, among which also 5000 V or 7000 V. such a document, therefore, explicitly specifies a possible usable value of voltage, explicating, however, that with such values is favoured exclusively the oxygenation in the tissues.

Naturally, the single adjustment of voltage to one of said values is not alone sufficient for the production of nitric oxide and the same text mentions such values as optimal ones just for the purposes of obtaining a greater oxygenation in the organism, in absolute accordance with the preceding document cited "LIANI".

Publication US2008/0275374 is also known, which describes an apparatus in the form of a bandaging which is suitable for the generation of an electrostatic field to favour a healing process where there is an injury. Such a type of bandage comprises two conductive plates separated by a dielectric and, through a controller, it is possible to charge them to inject a slight current. The document cites the injection of a current comprised between 300uA and 500 uA. Nevertheless, such a current is injected with the purpose of accelerating the healing process of injuries and no effect of production of nitric oxide is cited. Also in this case, the injection of the single current into said range is not able to cause the formation in the organism of nitric oxide.

The documents cited, therefore, describe setting parameters that, considered in an isolated manner, are not able to cause the production of nitric oxide and such settings are indicated and finalized to the production of oxygen or anyway for different scopes, without the mention of the problem relative to the production of nitric oxide.

### Disclosure of invention

It is therefore the aim of the present invention to provide an innovative device, and relative method, able to overcome, or at least mitigate significantly, said technical inconveniences.

In particular, it is the aim of the present invention to provide a device, and relative method, that stimulates in the human body in a natural way, and without the help of drugs, the production or the increase of production of nitric oxide.

These and other aims are therefore reached with the present device to stimulate the production of nitric oxide in the human body, as per claim 1.

Such device comprises:
- A voltage generator (V) connected to a conductive support surface (2).

According to the invention, the generator is programmed to generate a voltage comprised within a range, between 5Kv and 7Kv, with a circulating current in the human body comprised within a range between 0,1mA and 0,5mA.

In accordance with such functioning values, it has been surprisingly found out that the human organism is able to auto-generate a good quantity or optimize the production of nitric oxide without the help of drugs.

Such a solution overcomes the limits of the state of the art represented by devices which were notoriously programmed according to voltage and current values not idoneous at all to favour the production of nitric oxide but, on the contrary, to favour other processes such as the oxygenation of the tissues and the healing of injuries.

For example, as said, the setting suggested in De Lorenzo of the known New Health 9000 machine favours only and exclusively the production of oxygen and not of nitric oxide. The setting suggested in De Lorenzo with a possible voltage at 5000 V or 7000 V was not alone able to allow the production of nitric oxide in the body, because the combined effect of current comprised between 0,1mA and 0,5mA was missing and there was no indication in such publications of a possible combined setting of voltage-current to favour the production of nitric oxide.

Likewise, the American document US2008/0275374, which, above all, describes a device that acts locally in contrast to the New Health device that acts on the whole body, describes only a range of current usable in order to heal injuries and without discussing either the problem of production of the nitric oxide or any possible combination of voltage-current for the production of nitric oxide. There is no indication on the fact that such ranges of current could be combined with the voltage values used on the New Healt 9000 machine, above all used for different purposes, in order to favour the production of nitric oxide.

Advantageously, when the circulating current in the subject is maintained at a value of 0,1mA and with tension of 5000 Volts, then the effects of production of nitric oxide are further optimized.

A production of over the 10% can, in fact, be reached with respect to a basal situation of nitric oxide present in origin in the subject.

Advantageously, the frequency of the voltage is comprised between 50 Hz - 60 Hz, preferably at 50 Hz.

Advantageously, the support surface (2) can be a mat covered with conductive material or, alternatively, a support surface (2) can be foreseen in the form of an electrode in self-adhesive gel (hydrogel), preferably of a surface of 25 cm^2, covered with conductive material (for instance, like a cushion to be put on a chair).

Advantageously, a resistance that grounds is foreseen, in such a way as to maintain the circulating current in the range of values comprised between 0,1mA and 0,5mA, preferably at the value of 0,1mA.

It is also described here a method for stimulating the production of nitric oxide in the human body and comprising the arrangement of a voltage generator (V) and of a conductive support surface (2) connected to the voltage generator and characterized in that the voltage generator is made to operate within a range comprised between 5Kv and 7Kv with a circulating current in the subject comprised between 0,1mA and 0,5mA.

Advantageously, the current is at a value of 0,1mA and with tension of 5000 Volts.

### Brief description of drawings

Further features and advantages of the present device, and relative method, according to the invention, will result clearer with the description that follows of some embodiments, made to illustrate but not to limit, with reference to the annexed drawings, wherein:
- Figure 1 shows a schematization of the device in accordance with the present invention;
- Figure 2 shows a schematization with a patient lain on the device in accordance with the invention;
- Figure 3 shows a schematization with a patient seated on the device always in accordance with the invention;
- Figure 4 shows an electric scheme of the entire device.

### Description of some preferred embodiments

Apparatuses are known and commercialized by the same applicant that, through the generation of electrostatic fields, favour a phenomenon called "vasomotion", which are cyclical movements of the capillaries, favouring in that way the blood microcircle.

The greater blood supply, and therefore a better vascularization, has enormous beneficial effects on the whole organism, because that implies a better oxygenation for the tissues, favouring even the re-generation of the hair follicles for the hair.

The machinery commercialized is, for instance, visible on the Internet site:
http://www.akern.com/prodotti/new-health-9000.html

The machinery has the commercial name of "New Health 9000" and is constituted by a generator coupled to a ionizing mattress on which the patient is made to lie.

As literally shown on the site, New Health 9000 is a generator of high voltage electrostatic charges (up to 9 Kv) and very low current intensity, able to develop an electrostatic field on the surface of the body through a ionizing mattress. The field wraps the subject completely, stimulates the minerals in the blood and forms a thick layer of negative ions around the body that restores the surface charge of the red blood cells and that helps to normalize the blood pH.

The consequences of a normalization of the pH are: a greater supply of oxygen to the tissues; an increase of the metabolism; a normalization of the hydrostatic equilibrium; the improvement of the performance of the interstitial microenvironment.

Apart from that, the stimulation P.E.S.F. activates a contraction of the vasal walls with consequent increase of vascular mobility of the micro-circle as observed thanks to the analysis through laser-doppler.

The generator is able to generate a voltage V comprised within a range between 2.000 V ad 9000 V, generating a current of 0,1 mA at a frequency of the voltage of 50 +/- 60 hertz (basically the one of the house network).

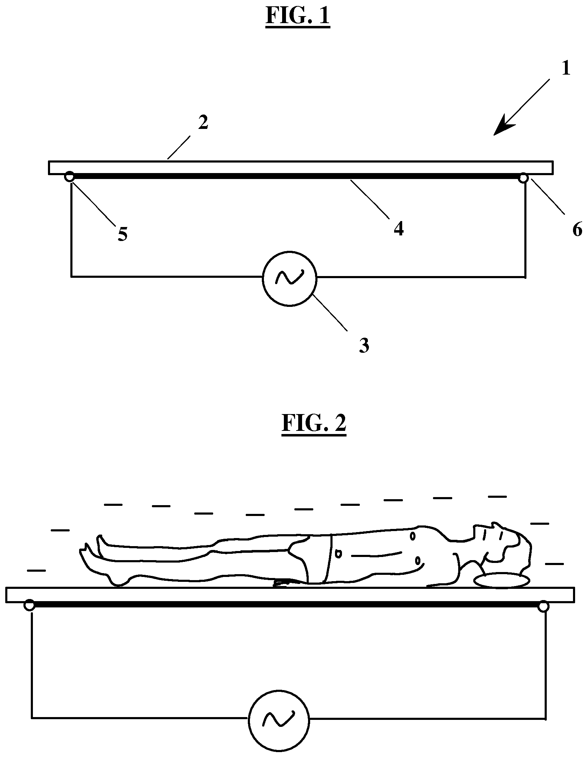

Figure 1 schematizes, therefore, the device through a schematization of the generator 3.

The generator 3 connects to the ionizing mat 2 constituted by a flexible sheet of plastic or fabric, rendered conductive through, for example, graphite or carbon varnishes. The conductive sheet is introduced in a pillowcase that is not necessarily conductive, washable or anyway replaceable due to hygiene needs.

As shown in figure 2, the patient is made to lie or seat on the ionized mat and said values of pulsed voltage and current generate an electrostatic field of negative charges that favour the motility or "vasomotion".

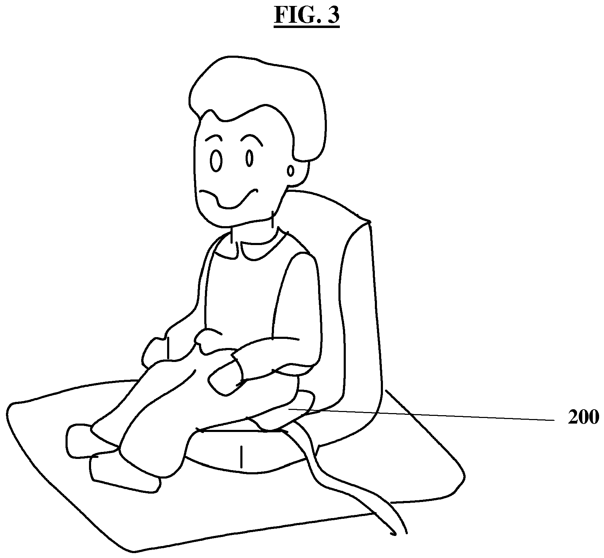

Likewise, as shown in figure 3, the patient can eventually be placed in a seated position.

In this case, leaving as it is that has been described so far, a normal chair and a cushion 200 are foreseen that replaces the mat and connects to the generator.

The whole is placed on an isolating surface in such a way that the patient lays the feet on said surface.

Having said that, according to the invention and leaving as it is that has been described, it has been surprisingly and unexpectedly found out that a predetermined range of functioning of the machinery determine and favour in the human body the natural production of nitric oxide.

In particular, a generator that generates a range of voltage comprised between 5Kv and 7Kv is needed, with a consequent circulating current in the subject that has to be maintained within a range between 0,1mA and 0,5mA.

This combined voltage-current effect allows to obtain the production of nitric oxide.

The frequency of such voltage is generally comprised between 50Hz and 60Hz.

With respect to the state of the art, therefore, it has been found out that this particular range, comprised within the greater range of 3Kv and 9Kv, allows the reaching of the desired technical effects, the stimulation of production of nitric oxide.

The current that is generated in the human body as a consequence, in accordance with such voltage, has to be maintained with a width comprised between 0,1mA and 0,5mA.

According to said voltage-current ranges, the organism subject to these voltage and current values succeeds in self-producing good quantities of nitric oxide, without having to resort to the use of drugs.

None of the documents of the state of the art cited discusses the problem of the production of nitric oxide and the possibility of obtaining a spontaneous production thereof in the organism through such a combination of voltage-current.

In general, it is preferred to maintain the current always at the specific value of 0,1mA.

The adjustment of the current is possible through a resistance that grounds and that eliminates the excess of current, maintaining it in the body at said value.

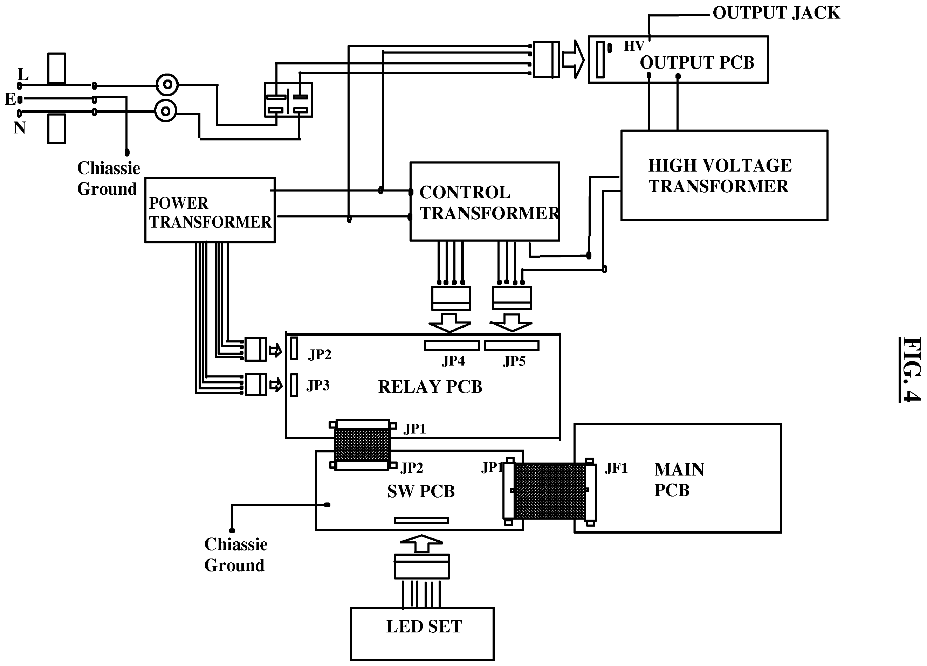

In that sense, the electric scheme of figure 4 shows such resistance that grounds the current in excess. The human body functions as a buffer system and a self-adjustment system is thus created so that the circulating current is always more or less of such value of 0,1 mA.

By varying the ground resistance and the voltage the circulating current can obviously be varied.

In particular, the current that circulates in the system kept constant at 0,1mA and with voltage of 5000 Volts and frequency of the voltage of 50 hertz has resulted to be particularly effective for the production of nitric oxide, optimizing to the maximum the production of the same.

Through, therefore, an increase in the natural production of nitric oxide is obtained without the need to have to use specific drugs.

It has been experimentally verified that, thanks to such a technology, it is possible to obtain an increase in the production of nitric oxide up to 10% more with respect to a basal situation of the human organism and with values that touch such a 10% in the case of voltage at 5000Vs and current at 0,1mA.

## Claims

1. A device for stimulating the production of nitric oxide in the human body and comprising:
- A voltage generator (V) connected to a conductive support surface (2);
- **Characterized in that** the generator is programmed to generate a voltage comprised between 5Kv and 7Kv, with a circulating current in the human body comprised between 0,1mA and 0,5mA.

2. A device, as per claim 1, wherein the current is at a value of 0,1mA and with a voltage of 5000 Volts.

3. A device, as per claim 1 or 2, wherein the frequency of the voltage is comprised between 50 Hz - 60 Hz.

4. A device, as per claim 1, wherein the support surface (2) is a mat covered with a conductive material.

5. A device, as per claim 1, wherein the support surface (2) is an electrode in self-adhesive gel (hydrogel), preferably of a surface of 25 cm^2, covered with a conductive material.

6. A device, as per one or more of the preceding claims, wherein a resistance is foreseen that discharges to the ground, in such a way as to maintain the circulating current in the range of values between 0,1mA and 0,5mA, preferably at the value of 0,1mA.
